# EUROPEAN PATENT SPECIFICATION

(11) **EP 4 166 152 B1**
(45) Date of publication and mention of the grant of the patent: **01.07.2026**
(21) Application number: 21822997.9
(22) Date of filing: 09.06.2021
(51) Int. Cl.: A61K 35/747, A23L 33/135, A61P 25/24

(54) **COMPOSITION FOR SUPPRESSING OR IMPROVING DEPRESSION**
ZUSAMMENSETZUNG ZUR UNTERDRÜCKUNG ODER VERBESSERUNG VON DEPRESSION
COMPOSITION DE SUPPRESSION OU D'AMÉLIORATION DE LA DÉPRESSION

(30) Priority: 10.06.2020 JP 2020101086
(43) Date of publication of application: 19.04.2023
(73) Proprietor: KIRIN HOLDINGS KABUSHIKI KAISHA, Nakano-ku, Tokyo 164-0001 (JP)
(72) Inventor: YAMAZAKI, Takahiro, Tokyo 164-0001 (JP); SUZUKI, Hiroaki, Tokyo 164-0001 (JP); MORITA, Yuji, Tokyo 164-0001 (JP)
(74) Representative: D Young & Co LLP
(86) International application number: PCT/JP2021/021899
(87) International publication number: WO 2021/251428

(56) References cited:
- WO-A1-2018/073963
- WO-A1-2019/121666
- JP-A- 2018 043 986
- US-A1- 2011 038 837
- WEI CHIA-LI; WANG SABRINA; YEN JUI-TING; CHENG YUN-FANG; LIAO CHIA-LI; HSU CHIH-CHIEH; WU CHIEN-CHEN; TSAI YING-CHIEH: "Antidepressant-like activities of live and heat-killedLactobacillus paracaseiPS23 in chronic corticosterone-treated mice and possible mechanisms", BRAIN RESEARCH, ELSEVIER, AMSTERDAM, NL, vol. 1711, 1 January 1900 (1900-01-01), NL , pages 202 - 213, XP085638087, ISSN: 0006-8993, DOI: 10.1016/j.brainres.2019.01.025

## Description

### TECHNICAL FIELD

The present invention relates to a composition for use in suppressing or improving depression.

### TECHNICAL FIELD

Depression is a state of loss of confidence such as feeling unworthy, feeling hopeless, or feeling guilty, and a stronger state of this state is referred to as depressive state. In addition, a depressive state which is severer than a certain level is referred to as clinical depression. Depression is a risk factor for clinical depression, is deeply related to declines in subjective well-being and life satisfaction, and adversely affects academic learning, work, and interpersonal relationship, and thus there is a need to prevent and improve depression.

There have been proposed so far a method for treating a depressive symptom using, as an active ingredient, bifidobacteria of Bifidobacterium longum as a food material (Patent Document 1), and a composition which suppresses a mood disorder associated with depression, comprising a saffron extract as an active ingredient (Patent Document 2).

### Reference List

### Patent Documents

Patent Document 1: JP 2018-525390 T
Patent Document 2: JP 2019-515919 T
WO 2019/121666 A1 relates to probiotics for cognitive and mental health.
Chia-Li et al., Brain Res. 2019 May 15;1711:202-213 relates to the antidepressant-like effects of Lactobascillus paracasei PS23.
WO 2018/073963 A1 relates to a mood profile improving agent comprising bacteria belonging to Lactobacillus paracasei.

### SUMMARY OF THE INVENTION

An object of the present invention is to provide a novel composition which suppresses or improves depression and an agent for suppressing or improving depression.

The present inventors have now found that, when depression was evaluated by a POMS2 inspection, depression before workload was significantly improved in test subjects who ingested Lactobacillus paracasei as compared with that in test subjects who did not ingest Lactobacillus paracasei. The present inventors have also found that, when depression was evaluated for test subjects who ingested Lactobacillus paracasei for four weeks or eight weeks, depression before workload was significantly improved as compared with that before ingestion. The present invention is based on these findings.

The present invention is set out in the appended set of claims.

Lactobacillus paracasei KW3110 strain, which is the active ingredient of the present invention, is a kind of lactic acid bacterium that has been traditionally eaten safely together with fermented foods. Therefore, the composition and agents of the present invention are advantageous in that they can be used as functional foods which suppress and/or improve depression, and that they can be used as safe functional foods for mammals including humans. According to the Treatment Guidelines II of the Japanese Society of Mood Disorders, the use of an antidepressant is not recommended for hypomelancholia, but the composition and agents of the present invention can be ingested daily as foods, and are advantageous in that they can be conveniently used to suppress and improve depression.

### DETAILED DESCRIPTION OF THE INVENTION

The present invention provides a composition and agents each comprising a lactic acid bacterium as an active ingredient. The lactic acid bacterium used as the active ingredient in the present invention is a lactic acid bacterium belonging to Lactobacillus paracasei (hereinafter sometimes referred to as the "lactic acid bacterium of the present invention"), and a Lactobacillus paracasei KW3110 strain (Lactobacillus paracasei, FERM BP-08634) is used.

As the lactic acid bacterium used in the present disclosure, a strain equivalent to the Lactobacillus paracasei KW3110 strain may be used. Here, the equivalent strain refers to a strain derived from the Lactobacillus paracasei KW3110 strain or a strain from which the Lactobacillus paracasei KW3110 strain is derived, or a progeny strain thereof.

The above-described Lactobacillus paracasei KW3110 strain has been deposited as FERM BP-08634 at the National Institute of Technology and Evaluation, Biotechnology Center, International Patent Organism Depositary (NITE-IPOD) (#120, 2-5-8 Kazusakamatari, Kisarazu, Chiba, 292-0818), which is the international depositary authority under the Budapest Treaty for deposit of patent microorganisms (deposit date: February 20, 2004). In addition, the strain derived from Lactobacillus paracasei KW3110 has been deposited as FERM BP-08635 at this International Patent Organism Depositary (deposit date: February 20, 2004).

The composition and agents of the present invention each preferably comprise either or both of a Lactobacillus paracasei cell and a treated product thereof as an active ingredient. The Lactobacillus paracasei cell may be in an isolated form or in a culture form.

Viable cells of Lactobacillus paracasei can be prepared by being cultured in a medium. Lactobacillus paracasei can be cultured by a known method using a known medium. As the medium, an M.R.S. medium, a GAM medium, or an LM17 medium can be used, and the medium may be added with an inorganic salt, a vitamin, an amino acid, an antibiotic, a serum or the like as appropriate and used. The culture may be performed at 25 to 40°C for several hours to several days. After the culture, the Lactobacillus paracasei cell is collected by centrifugation or filtration.

Examples of the treated product of the Lactobacillus paracasei cell include crushed products (e.g., ultrasonically crushed products) of viable cells or dead cells, dried products (e.g., freeze-dried products) of viable cells or dead cells, crushed products of the dried products, and enzymatically treated products of viable cells or dead cells. The dead cells can be obtained, for example, by heating treatment, treatment with a drug such as an antibiotic, treatment with a chemical substance such as formalin, treatment with ultraviolet rays, or treatment with radiation of gamma rays or the like. The enzymatically treated products include also treated products obtained by removing a cell wall of the lactic acid bacterium by an enzymatic or mechanical means. Further, nucleic acid-containing fractions (e.g., DNA and RNA) of Lactobacillus paracasei are also included in the treated product of the Lactobacillus paracasei cell, and can be obtained by dissolving the Lactobacillus paracasei cell with a surfactant or the like and then precipitating the cell with ethanol or the like. Hereinafter, the Lactobacillus paracasei cell and the treated product thereof, as used herein, are sometimes simply referred to as the "lactic acid bacterium of the present invention."

The composition and agents of the present invention can be each provided as the lactic acid bacterium of the present invention alone, or can be each provided as a mixture of the lactic acid bacterium of the present invention and other ingredients (for example, food raw materials, food additives, supplement raw materials, and/or formulation additives). The amount of the lactic acid bacterium of the present invention to be blended in the composition and agents of the present invention can be arbitrarily determined according to the purpose, intended use, form, dosage form, symptoms, body weight, and the like, and the content thereof can be 0.01 to 90% by mass, more preferably 0.1 to 50% by mass, based on the total amount, although the present invention is not limited thereto. In the present invention, the agents of the present invention can be designed so as to consist of the lactic acid bacterium of the present invention, and the composition of the present invention can be designed so as to comprise the lactic acid bacterium of the present invention and other ingredients.

As will be described in the Examples below, Lactobacillus paracasei has an effect for suppressing and improving depression. Therefore, the lactic acid bacterium of the present invention can be used as an active ingredient for suppressing and/or improving depression. The "depression," as used herein, means a state of loss of confidence such as feeling unworthy, feeling hopeless, or feeling guilty, and the meaning thereof includes dejection of mood. The meaning of the depression, as used herein, includes depression and dejection of mood that occur in daily life. In the present invention, the depression can be a condition which occurs in a person who is aware of eye fatigue.

In the present invention, the "suppression of depression" includes suppressing progression of depression and preventing depression. In addition, the "improvement of depression" includes alleviating depression and recovering from depression.

The suppression and/or improvement of depression in the present invention can be evaluated by a known method, and can be evaluated, for example, by Profile of Mood States 2nd Edition (short version) (in the present specification, sometimes referred to as "POMS2 inspection"). When the POMS2 inspection is used to evaluate depression, evaluation can be performed on the seven items "Anger-Hostility," "Confusion-Bewilderment," "Depression-Dejection," "Fatigue-Inertia," "Tension-Anxiety," "Vigor-Activity," "Friendliness", and "Total Mood Disturbance" in the POMS2 inspection. Depression can be evaluated using the score on the item "Depression-Dejection" as an index. Depression can be evaluated to have been suppressed and/or improved, when the score after ingestion was lowered as compared with that before ingestion in test subjects who ingested the composition and agents of the present invention, or when the score of the test subjects was lowered as compared with that of test subjects who did not ingest the composition or agents of the present invention.

The composition and agents of the present invention can be provided in the form of pharmaceutical products (e.g., pharmaceutical compositions), quasi-drugs, foods (e.g., food compositions), feeds (including pet foods) or the like, and can be implemented according to the following descriptions.

The active ingredient of the present invention can be orally administered to humans and non-human animals. Oral agents include granules, powders, tablets (including sugar-coated tablets), pills, capsule agents, syrups, emulsions and suspensions. These formulations can be formulated using a pharmaceutically acceptable carrier by a technique commonly carried out in the art. The pharmaceutically acceptable carrier includes excipients, binders, diluents, additives, perfumes, buffers, thickeners, colorants, stabilizers, emulsifiers, dispersants, suspending agents, and preservatives.

The active ingredient of the present invention can be orally fed to humans and non-human animals, and a typical ingestion form thereof is a food. When the active ingredient of the present invention is provided as a food, the active ingredient can be provided as it is as a food, or can be provided by being incorporated in a food. The thus-provided food is a food containing an effective amount of the active ingredient of the present invention. The phrase "containing an effective amount" of the active ingredient of the present invention, as used herein, refers to a content of the active ingredient of the present invention to be ingested, within a range as will be described later, when individual foods are ingested in a normally-eaten amount. The meaning of the "food" as used herein, includes health foods, functional foods, nutritional supplements, foods with health claims (such as foods for specified health uses, foods with nutrient function claims, and foods labeled with functions), foods for special dietary uses (such as foods for infants, foods for expectant and nursing mothers, and foods for sick persons) and supplements. When the active ingredient of the present invention is fed to an animal other than a human, needless to say, the food referred to herein is used as a feed.

The active ingredient of the present invention has the effect for suppressing and improving depression as described above, and thus can be contained in foods ingested daily, or can be provided as a supplement. In this case, the composition and agents of the present invention can be each provided in a unit package form in which the ingestion amount per meal is predetermined. Examples of the unit package form per meal include forms which define a constant amount using a pack, a package, a can, a bottle and the like. In order to allow the composition and agents of the present invention to exert various actions better, the ingestion amount per meal can be determined according to the daily ingestion amount of the active ingredient of the present invention which will be described later. The food of the present invention may be provided in the form of a package on which an explanation about the ingestion amount is given, or may be provided together with a document or the like which explains the ingestion amount.

The predetermined ingestion amount per meal in the unit package form may be either the effective daily ingestion amount or an ingestion amount obtained by dividing the effective daily ingestion amount into two or more (preferably two or three) portions. Thus, the unit package form of the composition and agents of the present invention can contain the active ingredient of the present invention in the daily ingestion amount for a human which will be described later, or can contain the active ingredient of the present invention in an amount half to one sixth of the daily ingestion amount for a human which will be described later. For convenience of ingestion, the composition and agents of the present invention are each preferably provided in the unit package form per meal (i.e., the unit package form per day) in which the ingestion amount per meal is the effective daily ingestion amount.

The form of the "food" is not particularly limited, and the food may be provided, for example, in a beverage form, in a semi-liquid or gelled form, or in a solid or powder form. The "supplement" includes tablets manufactured by adding an excipient, a binder and the like to the active ingredient of the present invention, kneading them together and then tableting the kneaded product, granules manufactured by adding an excipient, a binder and the like to the active ingredient of the present invention and granulating them, orally disintegrating tablets, and capsule agents in which the active ingredient of the present invention is encapsulated in a capsule and the like. When the composition and agents of the present invention are each provided as a supplement, it is also suitable to provide them not only in the above-described unit package form per meal or per day, but also in a unit package form per week, per two weeks, per month, or per two months. In the case of the latter unit package form, for example, it is desirable that the ingestion amount per meal or per day should be indicated so that the person who ingests the supplement himself/herself can ingest the effective amount of the active ingredient of the present invention according to the indication.

The food provided in the present invention is not particularly limited as long as it contains the active ingredient of the present invention, and examples thereof can include non-alcoholic beverages such as refreshing drinks, carbonated drinks, drinks containing fruit juice, drinks containing vegetable juice, drinks containing fruit juice and vegetable juice, animal milk such as cow milk, soybean milk, milk beverages, drink-type yogurt, drink-type and stick-type jellies, coffee, cocoa, tea drinks, nutritional drinks, energy drinks, sports drinks, mineral water, flavored water, and non-alcohol beer-taste beverages; carbohydrate-containing foods and beverages such as rice, noodles, bread and pasta; dairy products such as cheese, hard-type or soft-type yogurt, fresh cream composed of animal milk and other oil and fat raw materials, and ice cream; various confectioneries such as Western-style confectioneries including cookies, cakes and chocolate, Japanese-style confectioneries including buns with a bean-jam filling and sweet jellies of adzuki beans, tablet confectioneries (refreshing confectioneries) including soda pop-flavored sweets, candies, gums, gummies, chilled sweets and frozen sweets including jellies and puddings, and snacks; alcoholic beverages such as whiskey, bourbon, spirit, liqueur, wine, fruit wine, sake (Japanese rice wine), Chinese liquor, shochu (Japanese distilled spirit), beer, non-alcohol beer having an alcohol content of 1% or less, low-malt beer, other miscellaneous liquors and shochu mixed with soda water; processed products in which eggs are used, processed products of fish and meat (including giblets such as lever) (including rare delicacy), processed foods such as soup including miso soup, flavorings such as miso, soy sauce, rice seasoning and other seasonings, and liquid diets such as high density liquid diets. It should be noted that mineral water includes both of effervescent mineral water and non-effervescent mineral water. The food provided in the present invention contains both a food manufacturing raw material and a food additive.

Tea drinks include all of fermented tea, semi-fermented tea and unfermented tea, and examples thereof include black tea, green tea, barley tea, genmaicha (brown rice tea), sencha (middle-grade green tea), gyokurocha (refined green tea), hojicha (roasted green tea), oolong tea, turmeric herbal tea, Pu-erh tea, rooibos tea, rose tea, chrysanthemum tea, ginkgo leaf tea and herb tea (such as mint tea and jasmine tea).

Examples of fruits used in drinks containing fruit juice and drinks containing fruit juice and vegetable juice include apple, orange, grape, banana, pear, peach, mango, acai, blueberry and ume (plum). Examples of vegetables used in drinks containing vegetable juice and drinks containing fruit juice and vegetable juice include tomato, carrot, celery, pumpkin, cucumber and watermelon.

The ingestion amount of the active ingredient of the present invention can be determined depending on the recipient's sex, age and weight, symptoms, ingestion time, dosage form, ingestion route and drugs to be combined. The daily ingestion amount for an adult of the active ingredient of the present invention can be specified, for example, by the number of bacteria. A lower limit value of the ingestion amount can be 1×10⁷ cells (bacteria), 1×10⁸ cells, or 1×10⁹ cells, and an upper limit value thereof can be 1×10¹¹ cells, 1×10¹² cells, or 1×10¹³ cells. The upper and lower limit values can be combined arbitrarily, and the range of the injection amount can be, for example, 1×10⁷ to 1×10¹³ cells, 1×10⁸ to 1×10¹² cells, or 1×10⁹ to 1×10¹¹ cells. The number of lactic acid bacteria can be measured, for example, by fluorescent staining, flow cytometry, and cultivation.

The daily ingestion amount for an adult of the active ingredient of the present invention can also be specified by the dry cell mass. A lower limit value of the ingestion amount can be 1 mg, 10 mg or 25 mg, and an upper limit value thereof can be 1000 mg, 500 mg or 100 mg. The upper and lower limit values can be combined arbitrarily, and the range of the ingestion amount can be, for example, 1 to 1000 mg, 10 to 500 mg, or 25 to 100 mg.

The above-described ingestion amount of, and the below-described ingestion timing and ingestion period for the active ingredient of the present invention are applicable when the active ingredient of the present invention is used for both non-therapeutic and therapeutic purposes, and the ingestion can be read as administration in the case of therapeutic purposes. The composition and agents of the present invention can also be each fed to mammals other than humans (e.g., cows, horses, sheep, pigs, dogs and cats). The ingestion amount, ingestion timing and ingestion period can be determined with reference to the descriptions regarding humans.

The active ingredient of the present invention is preferably ingested continuously during a period when a depression suppression or improvement action is expected. The ingestion period for the active ingredient of the present invention, in the above-described daily amount, can be set to one week or longer, two weeks or longer, three weeks or longer, preferably one month or longer (four weeks or longer), from the viewpoint of providing the effect for suppressing and/or improving depression better. The ingestion interval for the active ingredient of the present invention, in the above-described daily amount, can be set to once every three days, once every two days or once a day, and is preferably once a day.

The ingestion of the active ingredient of the present invention may also be started before the date when the depression suppression or improvement action is expected. Examples of dates before the date when the depression suppression or improvement action is expected include dates before an event which tends to make a person feel blue and dates before an event which easily makes a person feel dejected or brings a person in a dejected mood.

The composition and agents as well as food of the present invention may be labeled as having an effect for suppressing and/or improving depression. In this case, the label can be designed so as to be easily understood by consumers. Examples of the label for these foods and beverages include, but are not limited to, some or all of the following indications:
· for persons who tend to feel blue;
· for persons who easily feel dejected;
· for persons who tend to feel down; and
· for persons who easily gloom.

According to yet another aspect of the present invention, there is provided Lactobacillus paracasei or a composition comprising Lactobacillus paracasei, for use in suppressing and/or improving depression, or for use in a method for suppressing and/or improving depression. The Lactobacillus paracasei described above can be carried out according to the descriptions about the composition and agents of the present invention.

The use of the present invention may be used in mammals including humans, and is intended to involve both of therapeutic use and non-therapeutic use. The "non-therapeutic," as used herein, means elimination of an activity of operating on, treating, or diagnosing a human (i.e., medical activity to a human), and specifically means elimination of a method of performing operation on, treatment of, or diagnosis involving a human by a doctor or a person who receives an instruction from the doctor.

### EXAMPLES

Hereinafter, the present invention will be described in more detail by way of the following examples, but is not limited thereto.

### Example 1: Depression-Dejection improvement effect of KW3110 strain

### (1) Outline of test

This test was examined and approved by an outside ethics committee, and conducted at a third-party institution. The test was a randomized, double-blind, placebo-controlled, parallel-group comparative test. Specifically, the test food or control food was fed to test subjects during a test period to confirm the effect of the KW3110 strain against depression.

### (2) Food to be tested

As the food to be tested, the test food "KW3110 strain-containing capsule" or the control food "KW3110 strain-free capsule" was used. During the test period (eight weeks), one capsule of the food to be tested (test food or control food) was fed to the test subjects together with water or lukewarm water every day once a day after breakfast. As indicated in Table 1, the test food contains 50 mg of a KW3110 strain heat-killed cell powder per capsule (number of bacteria: 10⁹ to 10¹¹ cells), and the control food contains 50 mg of corn starch instead of the KW3110 strain.

**[Table 1]**

| Table 1: Formulation of foods to be tested (per capsule) | | | | |
|---|---|---|---|---|
| Raw material name | Test food | | Control food | |
| | Mass (mg) | Blending rate (%) | Mass (mg) | Blending rate (%) |
| KW3110 strain (powder) | 50 | 25 | 0 | 0 |
| Corn starch (non-genetically modified) | 150 | 75 | 200 | 100 |
| Total | 200 | 100 | 200 | 100 |

### (3) Test subject

Healthy men and women of 35 years old and over and under 50 years old, who felt eye fatigue and had been determined to be healthy by a doctor in a preliminary examination, were used as the test subjects. The test subjects were randomly allocated to a group to which the test food was fed (hereinafter sometimes referred to as "test food group") and a group to which the control food was fed (hereinafter sometimes referred to as "control food group"). During the test period, the test subjects were allowed to continue a similar life to that before the test period. The breakdown of the target subjects for analysis was as indicated in Table 2.

**[Table 2]**

| Table 2: Breakdown of target subject for analysis | | |
|---|---|---|
| | Test food group | Control food group |
| Number of target subjects (subjects) | 40 (15/25) | 40 (13/27) |
| (men/women) | | |
| Average age (years) | 42.5 ± 3.9 | 42.4 ± 4.4 |

### (4) Load

A workload was applied to the test subjects by conducting the Uchida-Kraepelin test (Toyohiko Tonooka, Uchida-Kraepelin Psychodiagnostic Test·Basic Text, edited by Nisseiken Inc., 1973, pp. 1-16). The Uchida-Kraepelin test was conducted at each of their visits before the initiation of ingestion of the food to be tested, at Week 4 after the initiation of ingestion of the food to be tested, and at Week 8 after the initiation of ingestion of the food to be tested.

### (5) Measurement

### A. Measurement items

The measurement items were the items of the POMS2 inspection, which can evaluate the subjective states at measurement time points. The POMS2 inspection is an inspection suitable for evaluating states at the measurement time points and changes in effect of intervention over time for seven items: "Anger-Hostility," "Confusion-Bewilderment," "Depression-Dejection," "Fatigue-Inertia," "Tension-Anxiety," "Vigor-Activity" and "Friendliness."

### B. Measurement method

The POMS2 inspection was conducted to evaluate the test subjects' mood states at the measurement time points and the changes in effect of intervention over time. Specifically, the test subjects were asked to answer a total of 35 questions about their mood states. The answers were calculated for each of the seven items: "Anger-Hostility," "Confusion-Bewilderment," "Depression-Dejection," "Fatigue-Inertia," "Tension-Anxiety," "Vigor-Activity" and "Friendliness" to obtain measured values. A decrease in measured value means weakening of each mood state, and a decrease in measured value of "Depression-Dejection" means suppression and improvement of depression-dejection.

### C. Measurement timing

The POMS2 inspection was conducted before and after the workload and after 30-minute rest after the workload, the workload having been applied by conducting the Uchida-Kraepelin test at their visits before the initiation of ingestion of the food to be tested, at Week 4 after the initiation of ingestion of the food to be tested, and at Week 8 after the initiation of ingestion of the food to be tested.

### (6) Evaluation and analysis

### A. Method for evaluating numerical value

In the POMS2 inspection, scores at the respective measurement time points were obtained. The measured values at Week 4 after the initiation of ingestion of the food to be tested and at Week 8 after the initiation of ingestion thereof for both the groups were subjected to an assay using analysis of variance (ANOVA) with test participants and time points as fixed factors, as intra-group comparison. In addition, as inter-group comparison, interactions and main effects were evaluated using two-way repeated measures analysis of co-variance (ANCOVA) with the measured value before the initiation of ingestion of the food to be tested as a covariance and the time points and groups as factors. In both cases, an assay using the LSD method was conducted as a post-hoc assay.

### (7) Result

### A. Before workload

The results before the workload at each of the time points before the initiation of ingestion, at Week 4 after the initiation of ingestion, and at Week 8 after the initiation of ingestion were as indicated in Table 3.

**[Table 3]**

| Table 3: Evaluation results of "Depression-Dejection" in POMS2 inspection | | | |
|---|---|---|---|
| | Before ingestion | Week 4 | Week 8 |
| Test food group | 45.75 ± 6.46 | 43.58 ± 4.78*^{##} | 42.65 ± 4.32 ^{###} |
| Control food group | 46.08 ± 6.62 | 46.43 ± 8.00 | 44.43 + 5.10 |

| | | | |
|---|---|---|---|
| The values are indicated as average value ± standard deviation. Intra-group comparison ^{##}p<0.01, ^{###}p<0.001 (ANOVA with test participants and time points as fixed factors, LSD method as post-hoc assay) Inter-group comparison *p<0.05 (two-way repeated measures ANCOVA, LSD method as post-hoc assay) | | | |

From the results in Table 3, in terms of the item "Depression-Dejection" in the POMS2 inspection, the test food group improved significantly (p<0.05) as compared with the control food group at Week 4 after the initiation of ingestion, and improved with a significant trend (p<0.1) at Week 8 after the initiation of ingestion. In addition, when the scores at Week 4 and Week 8 after the initiation of ingestion were compared with those before the initiation of ingestion, the test food group showed a greater degree of improvement than the control food group. The test food group improved significantly (p<0.01) at Week 4 after the initiation of ingestion as compared with before the initiation of ingestion, and also improved significantly (p<0.001) at Week 8 after the initiation of ingestion. On the other hand, the control food group showed no significant improvement at Week 4 or Week 8 after the initiation of ingestion, as compared with before the initiation of ingestion. The improvement in depression before the workload is considered to indicate improvement in depression in daily life.

### B. Difference between before and after workload

A value (difference between before and after the workload) was determined by subtracting the value before the workload from the value after the workload at each of the time points, i.e., at the initiation of ingestion, at Week 4 after the initiation of ingestion, and at Week 8 after the initiation of ingestion. As a result, no difference was observed between the test food group and the control food group either at Week 4 after the initiation of ingestion or at Week 8 after the initiation of ingestion (data not shown).

### C. Difference between before and after rest

A value (difference between before and after rest) was determined by subtracting the value before the rest from the value after the rest at each of the time points, i.e., at the initiation of ingestion, at Week 4 after the initiation of ingestion, and at Week 8 after the initiation of ingestion. As a result, no difference was observed between the test food group and the control food group either at Week 4 after the initiation of ingestion or at Week 8 after the initiation of ingestion (data not shown).

### D. Conclusion

From the above results, the ingestion of the KW3110 strain-containing capsules significantly improved the score on the item "Depression-Dejection" of the test subjects in the POMS2 inspection, demonstrating that Lactobacillus paracasei has the effect for improving depression. In particular, the improvement before the workload was observed, demonstrating that Lactobacillus paracasei is effective in suppressing or improving depression in daily life.

## Claims

1. A composition for use in suppressing and/or improving depression, comprising Lactobacillus paracasei as an active ingredient, wherein the Lactobacillus paracasei is a Lactobacillus paracasei KW3110 strain (FERM BP-08634).

2. The composition for use according to claim 1, wherein the depression is a condition which occurs in a person who feels eye fatigue.

3. The composition for use according to claim 1 or 2, which is a food composition.

4. The composition for use according to any one of claims 1 to 3, which is a supplement.

5. The composition for use according to any one of claims 1 to 4, which is in a unit package form.

6. The composition for use according to any one of claims 1 to 5, which is provided to a human so that Lactobacillus paracasei is ingested in a daily amount of 1 mg or more in terms of dry cell mass.

7. The composition for use according to claim 6, which is ingested continuously for at least one week.

8. Lactobacillus paracasei or a composition comprising Lactobacillus paracasei, for use in suppressing and/or improving depression, or for use in a method for suppressing and/or improving depression, wherein the Lactobacillus paracasei is a Lactobacillus paracasei KW3110 strain (FERM BP-08634).

9. A food composition comprising Lactobacillus paracasei as an active ingredient for use in suppressing and/or improving depression, wherein the Lactobacillus paracasei is a Lactobacillus paracasei KW3110 strain (FERM BP-08634).

## Patentansprüche

1. Zusammensetzung zur Verwendung bei der Unterdrückung und/oder Verbesserung von Depression, umfassend *Lactobacillus paracasei* als Wirkstoff, wobei es sich bei den *Lactobacillus paracasei* um einen Lactobacillus-paracasei-KW3110-Stamm (FERM BP-08634) handelt.

2. Zusammensetzung zur Verwendung nach Anspruch 1, wobei es sich bei der Depression um ein Leiden handelt, das bei einer Person auftritt, die Augenermüdung fühlt.

3. Zusammensetzung zur Verwendung nach Anspruch 1 oder 2, bei der es sich um eine Lebensmittelzusammensetzung handelt.

4. Zusammensetzung zur Verwendung nach einem der Ansprüche 1 bis 3, bei der es sich um eine Ergänzung handelt.

5. Zusammensetzung zur Verwendung nach einem der Ansprüche 1 bis 4, die in einer Packungseinheitsform vorliegt.

6. Zusammensetzung zur Verwendung nach einem der Ansprüche 1 bis 5, die einem Menschen bereitgestellt wird, so dass *Lactobacillus paracasei* in einer Tagesmenge von 1 mg oder mehr, bezogen auf die Zelltrockenmasse, aufgenommen wird.

7. Zusammensetzung zur Verwendung nach Anspruch 6, die mindestens eine Woche lang kontinuierlich aufgenommen wird.

8. *Lactobacillus paracasei* oder eine Zusammensetzung, die *Lactobacillus paracasei* umfasst, zur Verwendung bei der Unterdrückung und/oder Verbesserung von Depression oder zur Verwendung bei einem Verfahren zur Unterdrückung und/oder Verbesserung von Depression, wobei es sich bei den *Lactobacillus paracasei* um einen Lactobacillus-paracasei-KW3110-Stamm (FERM BP-08634) handelt.

9. Lebensmittelzusammensetzung, umfassend *Lactobacillus paracasei* als Wirkstoff, zur Verwendung bei der Unterdrückung und/oder Verbesserung von Depression, wobei es sich bei den *Lactobacillus paracasei* um einen Lactobacillus-paracasei-KW3110-Stamm (FERM BP-08634) handelt.

## Revendications

1. Composition pour utilisation pour supprimer et/ou améliorer la dépression, comprenant Lactobacillus paracasei comme ingrédient actif, dans laquelle le Lactobacillus paracasei est une souche de Lactobacillus paracasei KW3110 (FERM BP-08634).

2. Composition pour utilisation selon la revendication 1, dans laquelle la dépression est un état qui se produit chez une personne qui ressent une fatigue oculaire.

3. Composition pour utilisation selon la revendication 1 ou 2, qui est une composition alimentaire.

4. Composition pour utilisation selon l'une quelconque des revendications 1 à 3, qui est un supplément.

5. Composition pour utilisation selon l'une quelconque des revendications 1 à 4, qui est sous une forme d'emballage unitaire.

6. Composition pour utilisation selon l'une quelconque des revendications 1 à 5, qui est fournie à un humain de sorte que Lactobacillus paracasei soit ingéré en une quantité quotidienne de 1 mg ou plus en termes de masse cellulaire sèche.

7. Composition pour utilisation selon la revendication 6, qui est ingérée en continu pendant au moins une semaine.

8. Lactobacillus paracasei ou composition comprenant Lactobacillus paracasei, pour une utilisation dans la suppression et/ou l'amélioration de la dépression, ou pour une utilisation dans un procédé de suppression et/ou d'amélioration de la dépression, dans lequel Lactobacillus paracasei est une souche de Lactobacillus paracasei KW3110 (FERM BP-08634).

9. Composition alimentaire comprenant Lactobacillus paracasei en tant qu'ingrédient actif destinée à être utilisée pour supprimer et/ou améliorer la dépression, dans laquelle Lactobacillus paracasei est une souche de Lactobacillus paracasei KW3110 (FERM BP-08634).
